# EUROPEAN PATENT APPLICATION

(11) **EP 4 190 778 A1**
(43) Date of publication of application: **07.06.2023**
(21) Application number: 21849867.3
(22) Date of filing: 21.07.2021
(51) Int. Cl.: C07D 405/14, C07D 409/14, H01L 51/00, H01L 51/50

(54) **HETEROCYCLIC COMPOUND AND ORGANIC LIGHT-EMITTING DEVICE COMPRISING SAME**

(30) Priority: 27.07.2020 KR 20200093407
(71) Applicant: LT Materials Co., Ltd., Yongin-si, Gyeonggi-do 17118 (KR)
(72) Inventor: CHOI, Eui-Jeong, Yongin-City, Gyeonggi-do 17118 (KR); NO, Young-Seok, Yongin-si, Gyeonggi-do 17118 (KR); PARK, Geon-Yu, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/009386
(87) International publication number: WO 2022/025516

(57) **Abstract**

The present application provides a heterocyclic compound capable of significantly enhancing lifetime, efficiency, electrochemical stability and thermal stability of an organic light emitting device, and an organic light emitting device comprising the heterocyclic compound in an organic material layer.

## Description

### [Technical Field]

This application claims priority to and the benefits of Korean Patent Application No. 10-2020-0093407, filed with the Korean Intellectual Property Office on July 27, 2020, the entire contents of which are incorporated herein by reference.

The present specification relates to a heterocyclic compound, an organic light emitting device comprising the same, and a composition for an organic material layer of an organic light emitting device.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

The present disclosure is directed to providing a heterocyclic compound, and an organic light emitting device comprising the same.

### [Technical Solution]

One embodiment of the present application provides a heterocyclic compound represented by the following Chemical Formula 1.

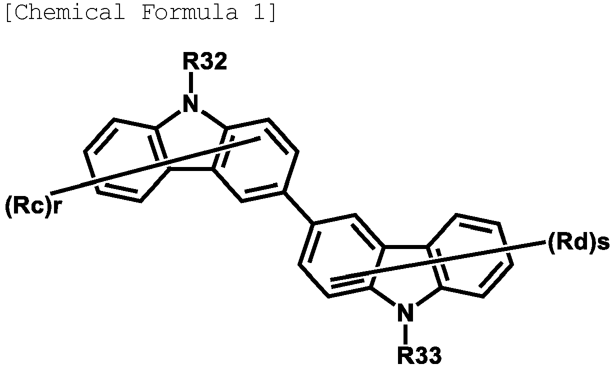

In Chemical Formula 1,
Rc and Rd are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 2 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, r and s are an integer of 0 to 7, and when r is 2 or greater, Rcs are the same as or different from each other, and when s is 2 or greater, Rds are the same as or different from each other,
R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms, and
the heterocyclic compound represented by Chemical Formula 1 has a deuterium content of greater than 0% and less than or equal to 100%.

Another embodiment of the present application provides an organic light emitting device comprising a first electrode, a second electrode, and one or more organic material layers provided between the first electrode and the second electrode, wherein one or more layers of the organic material layers comprise the heterocyclic compound represented by Chemical Formula 1.

### [Advantageous Effects]

A heterocyclic compound according to one embodiment of the present application can be used as a material of an organic material layer of an organic light emitting device. The heterocyclic compound can be used as a material of a hole injection layer, a hole transfer layer, a light emitting layer, an electron transfer layer, an electron injection layer, a charge generation layer and the like in an organic light emitting device. Particularly, the heterocyclic compound represented by Chemical Formula 1 can be used as a material of a light emitting layer of an organic light emitting device. In addition, using the heterocyclic compound represented by Chemical Formula 1 in an organic light emitting device is capable of lowering a driving voltage of the device, enhancing light efficiency, and enhancing lifetime properties of the device by thermal stability of the compound.

### [Description of Drawings]

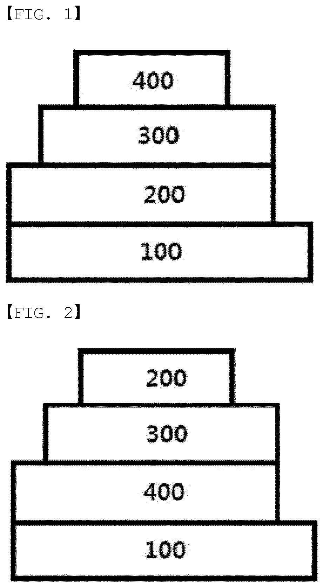

FIG. 1 to FIG. 3 are diagrams each schematically illustrating a lamination structure of an organic light emitting device according to one embodiment of the present application.

### [Mode for Disclosure]

Hereinafter, the present application will be described in detail.

One embodiment of the present application provides a heterocyclic compound represented by the following Chemical Formula 1.

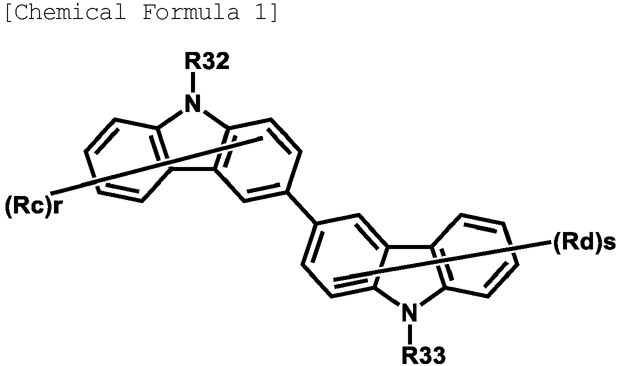

In Chemical Formula 1,
Rc and Rd are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 2 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, r and s are an integer of 0 to 7, and when r is 2 or greater, Rcs are the same as or different from each other, and when s is 2 or greater, Rds are the same as or different from each other,
R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms, and
the heterocyclic compound represented by Chemical Formula 1 has a deuterium content of greater than 0% and less than or equal to 100%.

A HOMO (highest occupied molecular orbital) level of the compound represented by Chemical Formula 1 is widely delocalized to two carbazoles. This increases HOMO level, hole mobility and stability compared to basic carbazole, and may enhance a lifetime of an organic light emitting device.

In addition, by having a structure that necessarily comprises deuterium, the compound substituted with deuterium having twice the atomic mass compared to hydrogen has decreased energy in a ground state and weakened intermolecular interactions due to lower zero-point energy and vibrational energy compared to compounds bonding to hydrogen, and as a result, a thin film may be made in a non-crystalline state and a device lifetime may be enhanced.

The deuterium-substituted compound has enhanced stability by having low energy in a ground state, and has enhanced molecular stability since dissociation energy of C-D is higher than dissociation energy of C-H, and a device using such a compound has an advantage of enhancing the lifetime.

In the present specification, the term "substitution" means a hydrogen atom bonding to a carbon atom of a compound being changed to another substituent, and the position of substitution is not limited as long as it is a position where the hydrogen atom is substituted, that is, a position where a substituent is capable of substituting, and when two or more substituents substitute, the two or more substituents may be the same as or different from each other.

In the present specification, the "substituted or unsubstituted" means being substituted with one or more substituents selected from the group consisting of deuterium; a cyano group; halogen; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; -P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 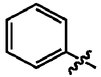 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

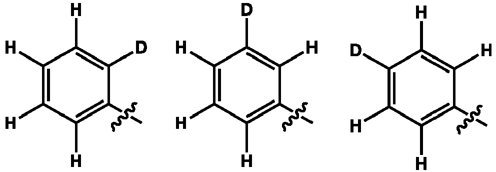

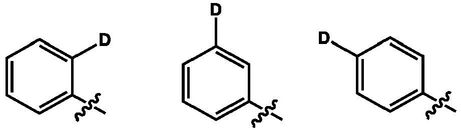

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide group may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

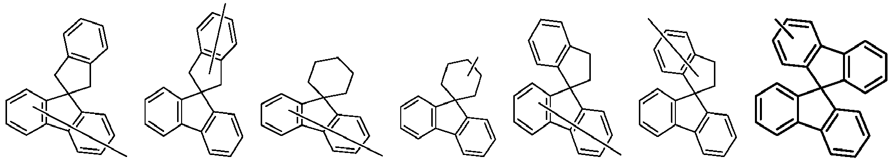

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto. In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except that these are each a divalent group. In addition, the heteroarylene group means the heteroaryl group having two bonding sites, that is, a divalent group. The descriptions on the heteroaryl group provided above may be applied thereto except that these are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In one embodiment of the present application, the heterocyclic compound represented by Chemical Formula 1 has a deuterium content of greater than 0% and less than or equal to 100%.

In one embodiment of the present application, the heterocyclic compound represented by Chemical Formula 1 has a deuterium content of greater than or equal to 10% and less than or equal to 100%.

In one embodiment of the present application, the heterocyclic compound represented by Chemical Formula 1 has a deuterium content of greater than or equal to 20% and less than or equal to 100%.

In one embodiment of the present application, the heterocyclic compound represented by Chemical Formula 1 has a deuterium content of greater than or equal to 40% and less than or equal to 100%.

In one embodiment of the present application, the deuterium content may be greater than or equal to 0% and less than or equal to 100% based on the positions where Rc and Rd may be substituted, and when the deuterium content is 0% based on the positions where Rc and Rd may be substituted, at least one of R32 and R33 may have a deuterium content of greater than 0% and less than or equal to 100%.

In one embodiment of the present application, the deuterium content is greater than 0% and less than or equal to 100% based on the positions where Rc and Rd may be substituted, and at least one of R32 and R33 may have a deuterium content of greater than or equal to 0% and less than or equal to 100%.

In one embodiment of the present application, the deuterium content is greater than 0% and less than or equal to 100% based on the positions where Rc and Rd may be substituted, and R32 and R33 may all have a deuterium content of 0%.

In one embodiment of the present application, the deuterium content is greater than 0% and less than or equal to 100% based on the positions where Rc and Rd may be substituted, and at least one of R32 and R33 may have a deuterium content of greater than 0% and less than or equal to 100%.

In one embodiment of the present application, the deuterium content is greater than 0% and less than or equal to 100% based on the positions where Rc and Rd may be substituted, and R32 and R33 may all have a deuterium content of greater than 0% and less than or equal to 100%.

In one embodiment of the present application, the deuterium content may be greater than or equal to 0% and less than or equal to 100% based on the position where Rc may be substituted, the deuterium content may be greater than or equal to 0% and less than or equal to 100% based on the position where Rd may be substituted, and when each deuterium content is 0% based on the positions where Rc and Rd may be substituted, at least one of R32 and R33 has a deuterium content of greater than 0% and less than or equal to 100%.

In one embodiment of the present application, the deuterium content may be greater than or equal to 0% and less than or equal to 100% based on the position where Rc may be substituted, the deuterium content may be greater than or equal to 0% and less than or equal to 100% based on the position where Rd may be substituted, and when a sum of the substituted deuterium content is greater than 0% based on the positions where Rc and Rd may be substituted, R32 and R33 may each independently have a deuterium content of greater than or equal to 0% and less than or equal to 100%.

In one embodiment of the present application, R32 and R33 may each independently have a deuterium content of greater than or equal to 0% and less than or equal to 100%, and when R32 and R33 all have a deuterium content of 0%, the deuterium content is greater than 0% and less than or equal to 100% based on the positions where Rc and Rd may be substituted.

In one embodiment of the present application, R32 and R33 each independently have a deuterium content of greater than or equal to 0% and less than or equal to 100%, and at least one of R32 and R33 may have a deuterium content of greater than 0%.

In one embodiment of the present application, R32 and R33 each independently have a deuterium content of greater than or equal to 0% and less than or equal to 100%, at least one of R32 and R33 has a deuterium content of greater than 0%, and the deuterium content may be 0% based on the positions where Rc and Rd may be substituted.

In one embodiment of the present application, R32 and R33 each independently have a deuterium content of greater than or equal to 0% and less than or equal to 100%, at least one of R32 and R33 has a deuterium content of greater than 0%, and the deuterium content may be greater than 0% and less than or equal to 100% based on the positions where Rc and Rd may be substituted.

In other words, the heterocyclic compound represented by Chemical Formula 1 necessarily comprises one or more deuterium.

In one embodiment of the present application, Rc and Rd are the same as or different from each other and may be each independently hydrogen; deuterium; or a substituted or unsubstituted aryl group having 6 to 60 carbon atoms, and the deuterium content may be greater than or equal to 0% and less than or equal to 100% based on the positions where Rc and Rd may be substituted.

In one embodiment of the present application, Rc and Rd are the same as or different from each other and may be each independently hydrogen; deuterium; or a substituted or unsubstituted aryl group having 6 to 40 carbon atoms, and the deuterium content may be greater than or equal to 0% and less than or equal to 100% based on the positions where Rc and Rd may be substituted.

In one embodiment of the present application, Rc and Rd are the same as or different from each other and may be each independently hydrogen; deuterium; or a substituted or unsubstituted aryl group having 6 to 20 carbon atoms, and the deuterium content may be greater than or equal to 0% and less than or equal to 100% based on the positions where Rc and Rd may be substituted.

In one embodiment of the present application, Rc and Rd are the same as or different from each other and may be each independently hydrogen; deuterium; a substituted or unsubstituted phenyl group; or a substituted or unsubstituted biphenyl group, and the deuterium content may be greater than or equal to 0% and less than or equal to 100% based on the positions where Rc and Rd may be substituted.

When the deuterium content is 0% based on the positions where Rc and Rd may be substituted, R32 and R33 each independently have a deuterium content of greater than 0% and less than or equal to 100%.

In one embodiment of the present application, Rc and Rd are the same as or different from each other and may be each independently hydrogen; deuterium; or a substituted or unsubstituted aryl group having 6 to 60 carbon atoms, and the deuterium content may be greater than 0% and less than or equal to 100% based on the positions where Rc and Rd may be substituted.

In one embodiment of the present application, Rc and Rd are the same as or different from each other and may be each independently hydrogen; deuterium; or a substituted or unsubstituted aryl group having 6 to 40 carbon atoms, and the deuterium content may be greater than 0% and less than or equal to 100% based on the positions where Rc and Rd may be substituted.

In one embodiment of the present application, Rc and Rd are the same as or different from each other and may be each independently hydrogen; deuterium; or a substituted or unsubstituted aryl group having 6 to 20 carbon atoms, and the deuterium content may be greater than 0% and less than or equal to 100% based on the positions where Rc and Rd may be substituted.

In one embodiment of the present application, Rc and Rd are the same as or different from each other and may be each independently hydrogen; deuterium; a substituted or unsubstituted phenyl group; or a substituted or unsubstituted biphenyl group, and the deuterium content may be greater than 0% and less than or equal to 100% based on the positions where Rc and Rd may be substituted.

In one embodiment of the present application, r and s are an integer of 2 to 7, and when r is 2 or greater, Rcs are the same as or different from each other, and when s is 2 or greater, Rds are the same as or different from each other. Two or more of Rcs are deuterium, and two or more of Rds are deuterium. At least two or more of each Rc and Rd are substituted.

In one embodiment of the present application, Rc and Rd are the same as or different from each other and may be each independently hydrogen; deuterium; or a substituted or unsubstituted aryl group having 6 to 60 carbon atoms, and two or more of Rcs are deuterium, and two or more of Rds are deuterium.

In one embodiment of the present application, Rc and Rd are the same as or different from each other and may be each independently hydrogen; deuterium; or a substituted or unsubstituted aryl group having 6 to 40 carbon atoms, and two or more of Rcs are deuterium, and two or more of Rds are deuterium.

In one embodiment of the present application, Rc and Rd are the same as or different from each other and may be each independently hydrogen; deuterium; or a substituted or unsubstituted aryl group having 6 to 20 carbon atoms, and two or more of Rcs are deuterium, and two or more of Rds are deuterium.

In one embodiment of the present application, Rc and Rd are the same as or different from each other and may be each independently hydrogen; deuterium; a substituted or unsubstituted phenyl group; or a substituted or unsubstituted biphenyl group, and two or more of Rcs are deuterium, and two or more of Rds are deuterium.

In one embodiment of the present application, Rc and Rd are the same as or different from each other and may be each independently hydrogen; deuterium; a phenyl group; or a biphenyl group, and two or more of Rcs are deuterium, and two or more of Rds are deuterium.

In one embodiment of the present application, Rc and Rd are deuterium.

In one embodiment of the present application, R32 and R33 are the same as or different from each other and may be each independently a substituted or unsubstituted silyl group; a substituted or unsubstituted aryl group having 6 to 60 carbon atoms; or a substituted or unsubstituted heteroaryl group having 2 to 60 carbon atoms, and R32 and R33 each independently have a deuterium content of greater than or equal to 0% and less than or equal to 100%.

In one embodiment of the present application, R32 and R33 are the same as or different from each other and may be each independently a substituted or unsubstituted silyl group; a substituted or unsubstituted aryl group having 6 to 40 carbon atoms; or a substituted or unsubstituted heteroaryl group having 2 to 40 carbon atoms, and R32 and R33 each independently have a deuterium content of greater than or equal to 0% and less than or equal to 100%.

In one embodiment of the present application, R32 and R33 are the same as or different from each other and may be each independently a substituted or unsubstituted silyl group; a substituted or unsubstituted aryl group having 6 to 20 carbon atoms; or a substituted or unsubstituted heteroaryl group having 2 to 20 carbon atoms, and R32 and R33 each independently have a deuterium content of greater than or equal to 0% and less than or equal to 100%.

In one embodiment of the present application, R32 and R33 are the same as or different from each other and may be each independently a substituted or unsubstituted silyl group; a substituted or unsubstituted phenyl group; a substituted or unsubstituted biphenyl group; a substituted or unsubstituted terphenyl group; a substituted or unsubstituted triphenyl group; a substituted or unsubstituted naphthyl group; a substituted or unsubstituted dibenzothiophene group; or a substituted or unsubstituted dibenzofuran group, and R32 and R33 each independently have a deuterium content of greater than or equal to 0% and less than or equal to 100%.

In one embodiment of the present application, R32 and R33 each have a deuterium content of 100%, and Rc and Rd each have a deuterium content of 0%.

In one embodiment of the present application, R32 and R33 each have a deuterium content of 0%, and Rc and Rd each have a deuterium content of 100%.

In one embodiment of the present application, R32, R33, Rc and Rd each have a deuterium content of 100%. In other words, the heterocyclic compound represented by Chemical Formula 1 has a deuterium content of 100%.

In one embodiment of the present application, R32 and R33 have a deuterium content of 0%.

In one embodiment of the present application, Rc and Rd are deuterium, and R32 and R33 have a deuterium content of 0%.

In one embodiment of the present application, R32 and R33 are the same as or different from each other and each independently a substituted or unsubstituted silyl group; a substituted or unsubstituted aryl group having 6 to 60 carbon atoms; or a substituted or unsubstituted heteroaryl group having 2 to 60 carbon atoms, and R32 and R33 have a deuterium content of 0%.

In one embodiment of the present application, R32 and R33 are the same as or different from each other and each independently a substituted or unsubstituted silyl group; a substituted or unsubstituted aryl group having 6 to 40 carbon atoms; or a substituted or unsubstituted heteroaryl group having 2 to 40 carbon atoms, and R32 and R33 have a deuterium content of 0%.

In one embodiment of the present application, R32 and R33 are the same as or different from each other and each independently a substituted or unsubstituted silyl group; a substituted or unsubstituted aryl group having 6 to 20 carbon atoms; or a substituted or unsubstituted heteroaryl group having 2 to 20 carbon atoms, and R32 and R33 have a deuterium content of 0%.

In one embodiment of the present application, R32 and R33 are the same as or different from each other and each independently a substituted or unsubstituted silyl group; a substituted or unsubstituted phenyl group; a substituted or unsubstituted biphenyl group; a substituted or unsubstituted terphenyl group; a substituted or unsubstituted triphenyl group; a substituted or unsubstituted naphthyl group; a substituted or unsubstituted dibenzothiophene group; or a substituted or unsubstituted dibenzofuran group, and R32 and R33 have a deuterium content of 0%.

According to one embodiment of the present application, Chemical Formula 1 may be represented by any one of the following compounds, but is not limited thereto.

| | | | |
|---|---|---|---|
| 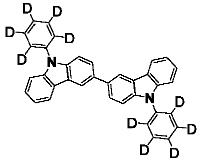 | 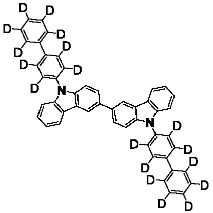 | 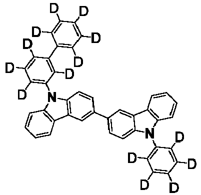 | 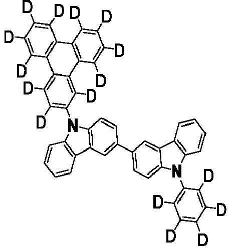 |
| 1-1 | 1-2 | 1-3 | 1-4 |
| 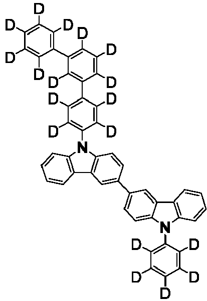 | 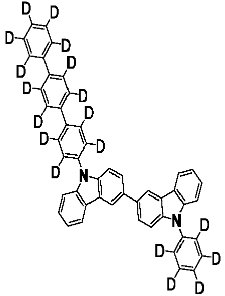 | 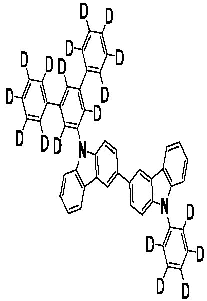 | 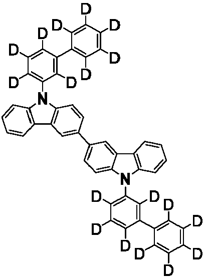 |
| 1-5 | 1-6 | 1-7 | 1-8 |
| 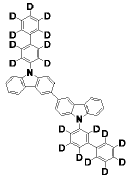 | 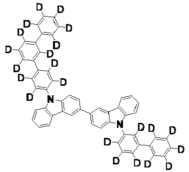 | 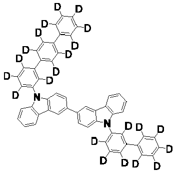 | 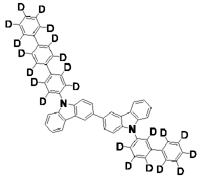 |
| 1-9 | 1-10 | 1-11 | 1-12 |
| 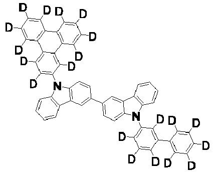 | 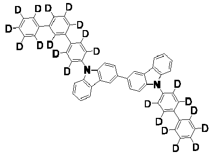 | 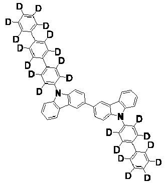 | 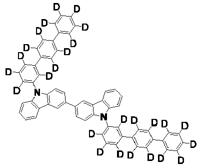 |
| 1-13 | 1-14 | 1-15 | 1-16 |
| 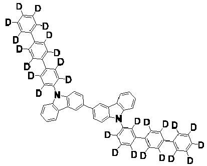 | 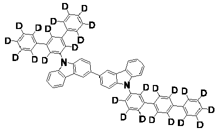 | 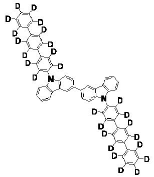 | 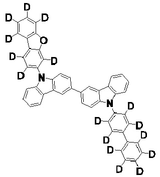 |
| 1-17 | 1-18 | 1-19 | 1-20 |
| 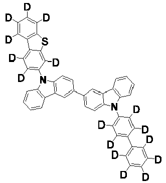 | 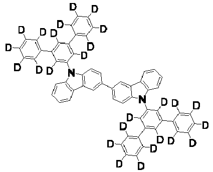 | 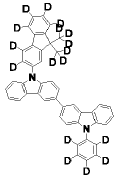 | 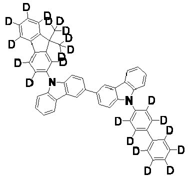 |
| 1-21 | 1-22 | 1-23 | 1-24 |
| 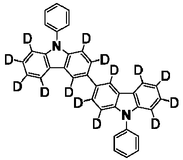 | 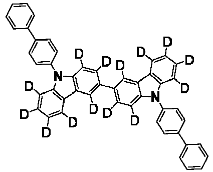 | 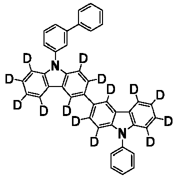 | 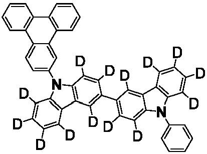 |
| 1-25 | 1-26 | 1-27 | 1-28 |
| 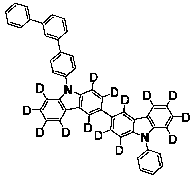 | 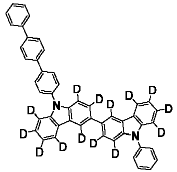 | 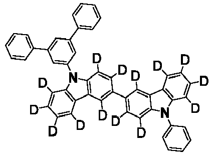 | 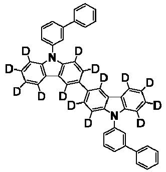 |
| 1-29 | 1-30 | 1-31 | 1-32 |
| 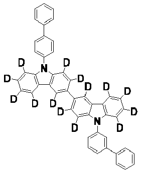 | 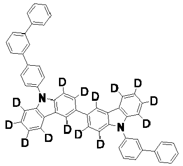 | 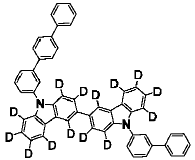 | 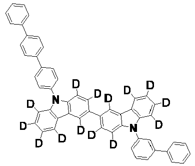 |
| 1-33 | 1-34 | 1-35 | 1-36 |
| 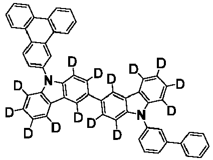 | 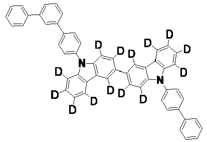 | 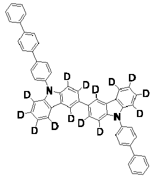 | 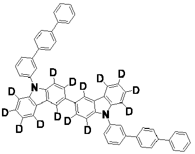 |
| 1-37 | 1-38 | 1-39 | 1-40 |
| 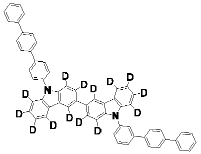 | 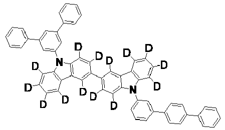 | 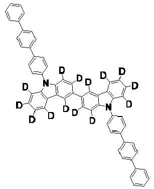 | 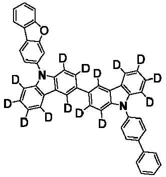 |
| 1-41 | 1-42 | 1-43 | 1-44 |
| 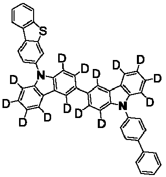 | 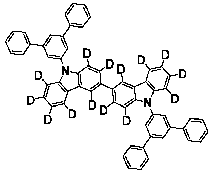 | 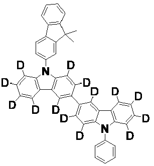 | 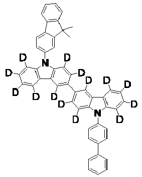 |
| 1-45 | 1-46 | 1-47 | 1-48 |
| 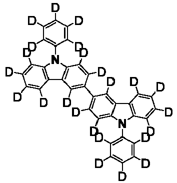 | 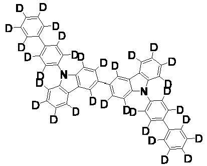 | 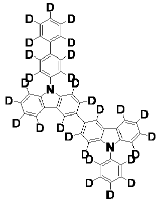 | 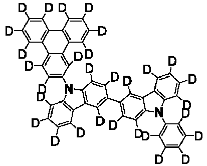 |
| 1-49 | 1-50 | 1-51 | 1-52 |
| 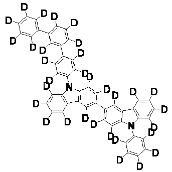 | 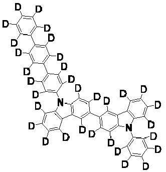 | 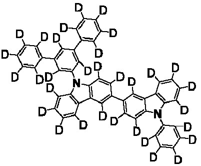 | 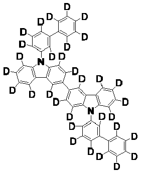 |
| 1-53 | 1-54 | 1-55 | 1-56 |
| 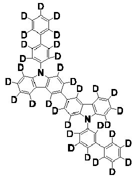 | 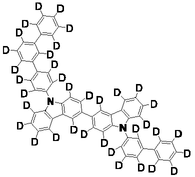 | 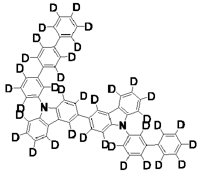 | 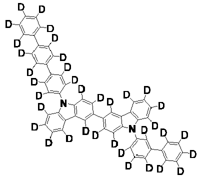 |
| 1-57 | 1-58 | 1-59 | 1-60 |
| 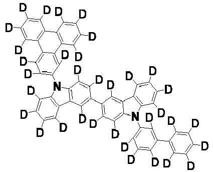 | 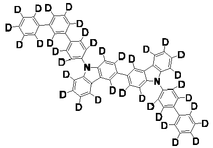 | 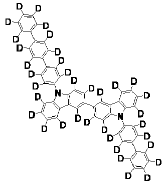 | 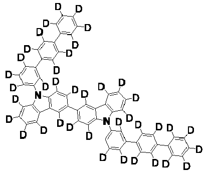 |
| 1-61 | 1-62 | 1-63 | 1-64 |
| 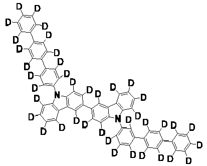 | 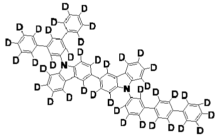 | 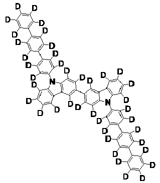 | 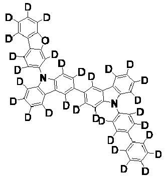 |
| 1-65 | 1-66 | 1-67 | 1-68 |
| 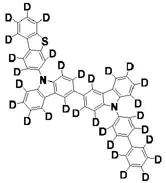 | 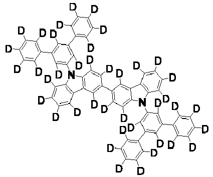 | 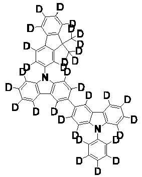 | 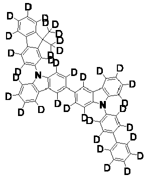 |
| 1-69 | 1-70 | 1-71 | 1-72 |
| 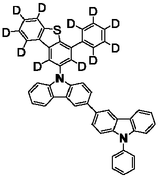 | 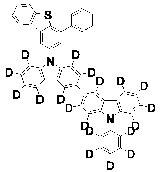 | 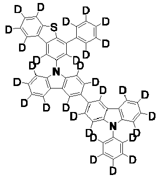 | 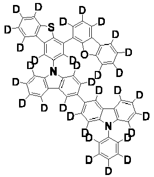 |
| 1-73 | 1-74 | 1-75 | 1-76 |

The heterocyclic compound according to one embodiment of the present application is represented by Chemical Formula 1. More specifically, the heterocyclic compound represented by Chemical Formula 1 may be used as a material of an organic material layer of an organic light emitting device due to such a core structure and structural properties of the substituents.

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the heterocyclic compound has a high glass transition temperature (Tg), and has excellent thermal stability. Such an increase in the thermal stability becomes an important factor providing driving stability to a device.

The heterocyclic compound according to one embodiment of the present application may be prepared using a multi-step chemical reaction. Some intermediate compounds are prepared first, and from the intermediate compounds, the heterocyclic compound of Chemical Formula 1 may be prepared. More specifically, the heterocyclic compound according to one embodiment of the present application may be prepared based on preparation examples to describe later.

Another embodiment of the present application provides an organic light emitting device comprising the heterocyclic compound represented by Chemical Formula 1. The "organic light emitting device" may be expressed in terms such as an "organic light emitting diode", an "OLED", an "OLED device" and an "organic electroluminescent device".

The heterocyclic compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

Specifically, the organic light emitting device according to one embodiment of the present application comprises a first electrode, a second electrode, and one or more organic material layers provided between the first electrode and the second electrode, and one or more layers of the organic material layers comprise the heterocyclic compound represented by Chemical Formula 1. When comprising the heterocyclic compound represented by Chemical Formula 1 in the organic material layer, the organic light emitting device has superior light emission efficiency and lifetime.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device.

In one embodiment of the present application, the organic light emitting device may be a green organic light emitting device, and the heterocyclic compound according to Chemical Formula 1 may be used as a material of the green organic light emitting device.

In one embodiment of the present application, the organic light emitting device may be a white organic light emitting device, and the heterocyclic compound according to Chemical Formula 1 may be used as a material of the white organic light emitting device.

In one embodiment of the present application, the organic light emitting device may be a red organic light emitting device, and the heterocyclic compound according to Chemical Formula 1 may be used as a material of the red organic light emitting device.

In addition, the organic material layer comprises one or more light emitting layers, and the light emitting layer comprises the heterocyclic compound represented by Chemical Formula 1. When comprising the heterocyclic compound represented by Chemical Formula 1 in the light emitting layer among the organic material layers, the organic light emitting device has more superior light emission efficiency and lifetime.

In the organic light emitting device of the present application, the organic material layer comprises a light emitting layer, and the light emitting layer may comprise the heterocyclic compound as a host material of a light emitting material.

The organic light emitting device of the present disclosure may further comprise one, two or more layers selected from the group consisting of a light emitting layer, a hole injection layer, a hole transfer layer, an electron injection layer, an electron transfer layer, a hole auxiliary layer and a hole blocking layer.

The organic light emitting device according to one embodiment of the present application may be manufactured using common organic light emitting device manufacturing methods and materials except that the organic material layer is formed using the heterocyclic compound described above.

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

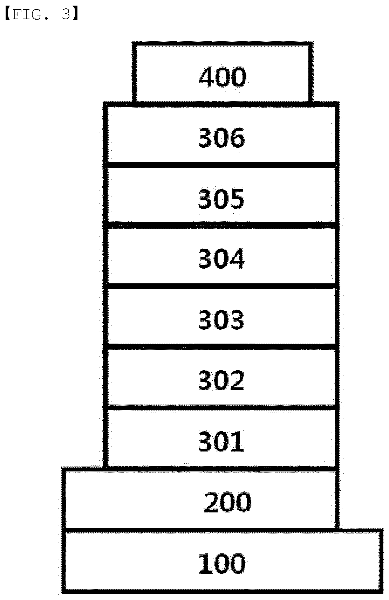

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

The organic light emitting device according to one embodiment of the present application may be a top-emission type, a bottom-emission type or a dual-emission type depending on the materials used.

The heterocyclic compound according to one embodiment of the present application may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Compound 1-2(B)

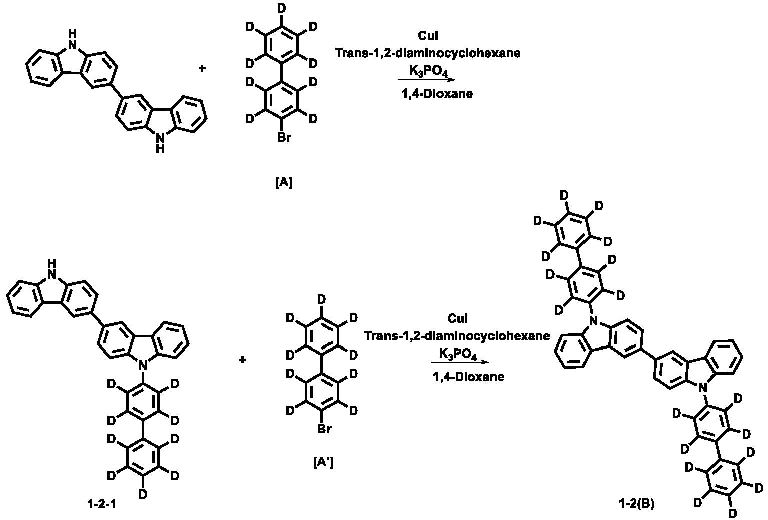

### 1) Preparation of Intermediate 1-2-1

In a one-neck round bottom flask, 9H,9'H-3,3'-bicarbazole (10 g, 0.030 mol), 4-bromo-1,1'-biphenyl-2,2',3,3',4',5,5',6,6'-D₉ [A] (7.26 g, 0.030 mol), CuI (0.57 g, 0.003 mol), trans-1,2-diaminocyclohexane (0.34 g, 0.003 mol) and K₃PO₄ (12.74 g, 0.06 mol) were dissolved in 1,4-dioxane (100 mL), and refluxed for 8 hours at 125°C. After the reaction was completed, the result was extracted by introducing distilled water and dichloromethane (hereinafter, also referred to as DCM) thereto at room temperature, and after drying the organic layer with MgSO₄, the solvent was removed using a rotary evaporator. The reaction material was purified by column chromatography (DCM:hexane=1:3), and recrystallized with methanol to obtain Intermediate 1-2-1 (13.92 g, yield 94%).

### 2) Preparation of Compound 1-2

In a one-neck round bottom flask, Intermediate 1-2-1 (13.92 g, 0.028 mol), 4-bromo-1,1'-biphenyl-2,2',3,3',4',5,5',6,6'-D₉ [A'] (6.83 g, 0.028 mol), CuI (0.53 g, 0.0028 mol), trans-1,2-diaminocyclohexane (0.32 g, 0.0028 mol) and K₃PO₄ (11.89 g, 0.056 mol) were dissolved in 1,4-dioxane (140 mL), and refluxed for 8 hours at 125°C. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain target Compound 1-2 (16.14 g, yield 88%).

When Compound A and Compound A' are the same, 2 equivalents of Compound A may be introduced in Preparation Example 1 to directly synthesize the target compound. In other words, when Compound A and Compound A' are the same, Preparation Example 1-2 (Preparation of Compound 1-2-1) may be skipped.

Target Compound B of the following Table 1 was synthesized in the same manner as in Preparation Example 1 except that Compounds A and A' of the following Table 1 were respectively used instead of 4-bromo-1,1'-biphenyl-2,2',3,3',4',5,5',6,6'-D₉ [A] and 4-bromo-1,1'-biphenyl-2,2',3,3',4',5,5',6,6'-D₉ [A'].

**[Table 1]**

| Compou nd | A | A' | B | Yield |
|---|---|---|---|---|
| 1-2 | 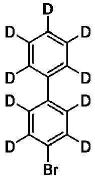 | 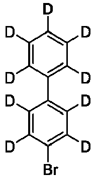 | 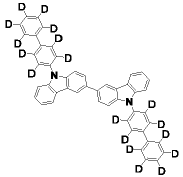 | 88% |
| 1-3 | 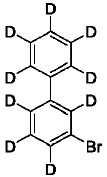 | 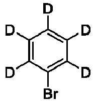 | 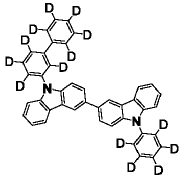 | 95% |
| 1-5 | 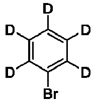 | 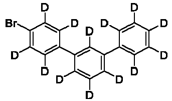 | 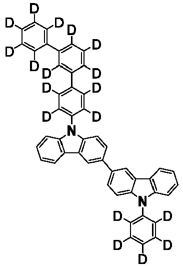 | 84% |
| 1-6 | 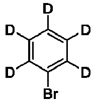 | 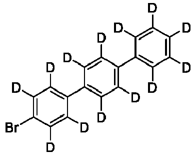 | 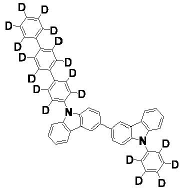 | 67% |
| 1-8 | 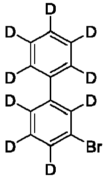 | 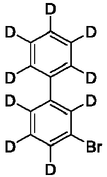 | 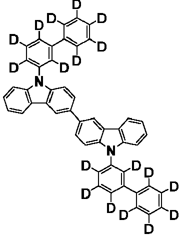 | 83% |
| 1-19 | 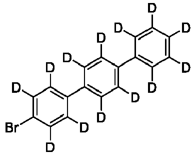 | 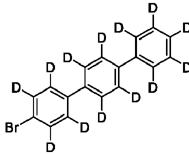 | 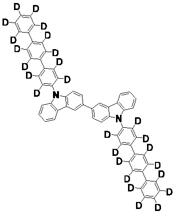 | 57% |
| 1-22 | 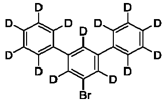 | 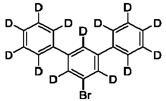 | 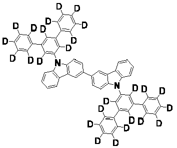 | 68% |

### <Preparation Example 2> Preparation of Compound 1-26(D)

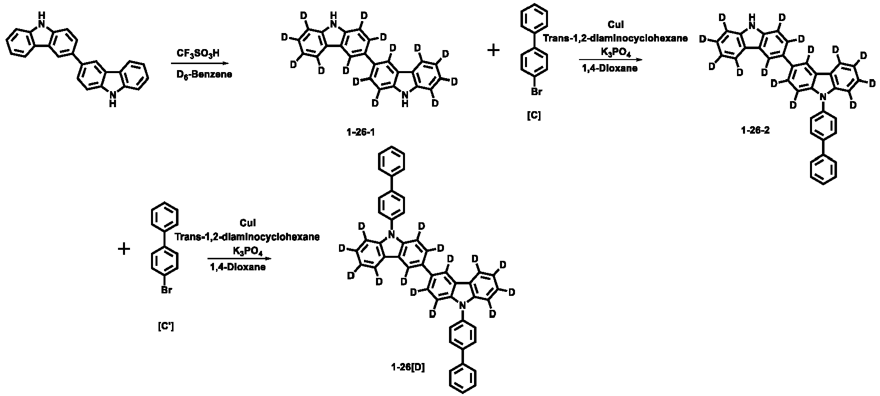

### 1) Preparation of Intermediate 1-26-1

In a one-neck round bottom flask, a mixture of 9H,9'H-3,3'-bicarbazole (10 g, 0.030 mol), triflic acid (112.56 g, 0.75 mol) and D₆-benzene (500 mL) was refluxed at 40°C. The result was quenched and extracted with DMC and H₂O, concentrated, and then silica gel filtered. The result was concentrated, and then treated with methanol to obtain Intermediate 1-26-1 (7.07 g, yield 68%) .

### 2) Preparation of Intermediate 1-26-2

In a one-neck round bottom flask, Intermediate 1-26-1 (7.07 g, 0.02 mol), CuI (0.38 g, 0.002 mol), 4-bromo-1,1'-biphenyl [C] (4.66 g, 0.02 mol), trans-1,2-diaminocyclohexane (0.23 g, 0.002 mol) and K₃PO₄ (8.49 g, 0.04 mol) were dissolved in 1,4-dioxane (70 mL), and refluxed for 8 hours at 125°C. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain Intermediate 1-26-2 (8.28 g, yield 83%).

### 3) Preparation of Compound 1-26

In a one-neck round bottom flask, Intermediate 1-26-2 (8.28 g, 0.017 mol), CuI (0.32 g, 0.0017 mol), 4-bromo-1,1'-biphenyl [C'] (3.96 g, 0.017 mol), trans-1,2-diaminocyclohexane (0.19 g, 0.0017 mol) and K₃PO₄ (7.22 g, 0.034 mol) were dissolved in 1,4-dioxane (80 mL), and refluxed for 8 hours at 125°C. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain target Compound 1-26 (8.63 g, yield 78%).

When Compound C and Compound C' are the same, 2 equivalents of Compound C may be introduced in Preparation Example 2-2 to directly synthesize the target compound. In other words, when Compound C and Compound C' are the same, Preparation Example 2-3 (Preparation of Compound 1-26-2) may be skipped.

Target Compound D of the following Table 2 was synthesized in the same manner as in Preparation Example 2 except that Compounds C and C' of the following Table 2 were respectively used instead of 4-bromo-1,1'-biphenyl [C] and 4-bromo-1,1'-biphenyl [C'].

**[Table 2]**

| Compou nd | C | C' | D | Yield |
|---|---|---|---|---|
| 1-26 | 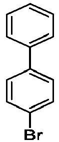 | 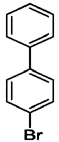 | 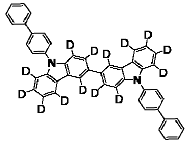 | 78% |
| 1-27 | 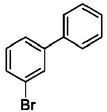 | 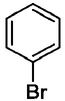 | 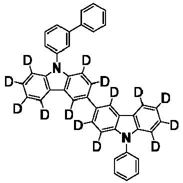 | 90% |
| 1-29 | 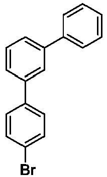 | 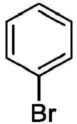 | 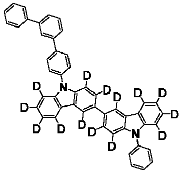 | 74% |
| 1-32 | 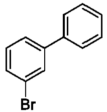 | 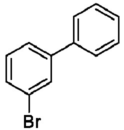 | 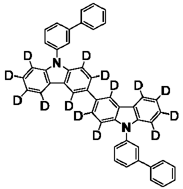 | 69% |
| 1-33 | 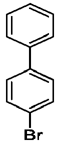 | 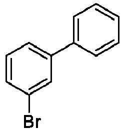 | 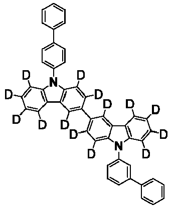 | 93% |
| 1-36 | 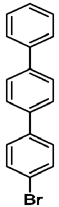 | 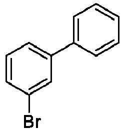 | 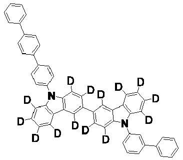 | 78% |
| 1-39 | 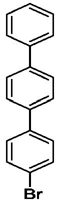 | 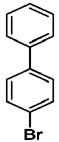 | 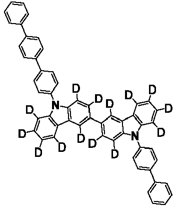 | 69% |
| 1-44 | 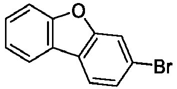 | 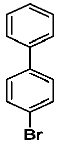 | 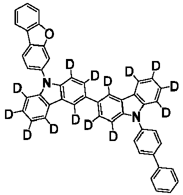 | 82% |

### <Preparation Example 3> Preparation of Compound 1-50(G)

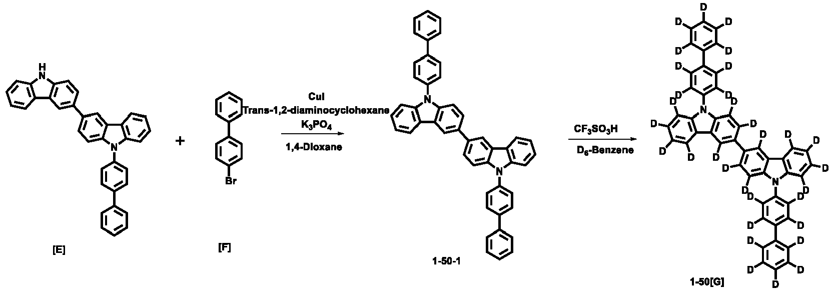

### 1) Preparation of Intermediate 1-50-1

In a one-neck round bottom flask, 9-([1,1'-biphenyl]-4-yl)-9H,9'H-3,3'-bicarbazole [E] (10 g, 0.021 mol), 4-bromo-1,1'-biphenyl [F] (4.90 g, 0.021 mol), CuI (0.40 g, 0.0021 mol), trans-1,2-diaminocyclohexane (0.024 g, 0.0021 mol) and K₃PO₄ (8.92 g, 0.042 mol) were dissolved in 1,4-dioxane (100 mL), and refluxed for 8 hours at 125°C. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain Intermediate 1-50-1 (12.17 g, yield 91%).

### 2) Preparation of Compound 1-50

In a one-neck round bottom flask, a mixture of Intermediate 1-50-1 (12.17 g, 0.017 mol), triflic acid (63.78 g, 0.43 mol) and D₆-benzene (600 mL) was refluxed at 50°C. The result was quenched and extracted with DMC and H₂O, concentrated, and then silica gel filtered. The result was concentrated, and then treated with methanol to obtain target Compound 1-50 (8.87 g, yield 78%).

Target Compound G of the following Table 3 was synthesized in the same manner as in Preparation Example 3 except that Compounds E and F of the following Table 3 were respectively used instead of 9-([1,1'-biphenyl]-4-yl)-9H,9'H-3,3'-bicarbazole [E] and 4-bromo-1,1'-biphenyl [F].

**[Table 3]**

| Compou nd | E | F | G | Yield |
|---|---|---|---|---|
| 1-50 | 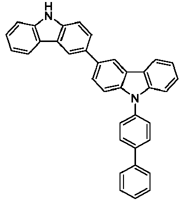 | 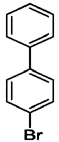 | 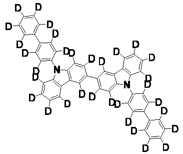 | 78% |
| 1-51 | 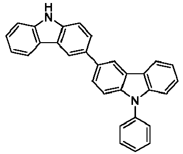 | 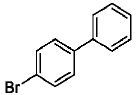 | 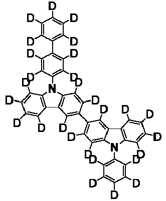 | 83% |
| 1-53 | 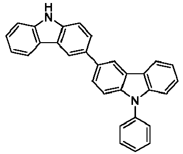 | 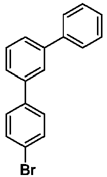 | 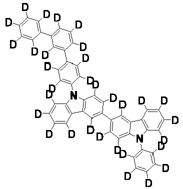 | 81% |
| 1-56 | 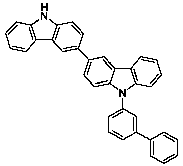 | 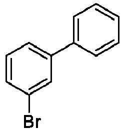 | 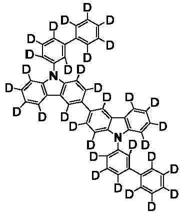 | 76% |
| 1-57 | 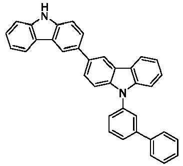 | 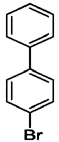 | 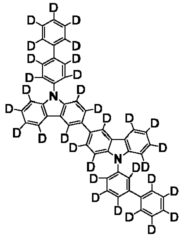 | 78% |
| 1-58 | 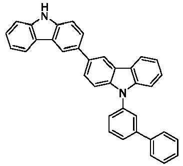 | 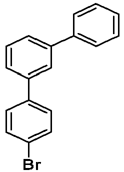 | 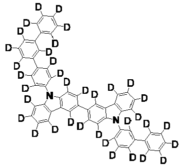 | 83% |
| 1-60 | 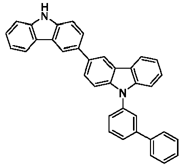 | 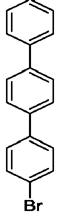 | 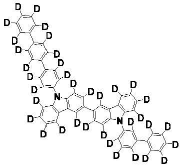 | 72% |
| 1-62 | 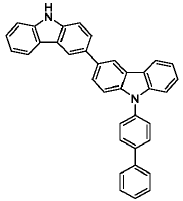 | 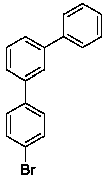 | 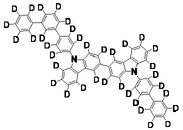 | 82% |
| 1-63 | 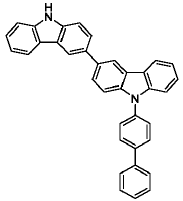 | 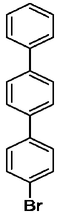 | 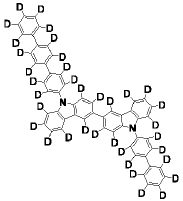 | 76% |
| 1-67 | 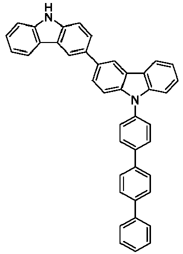 | 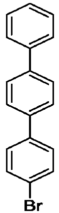 | 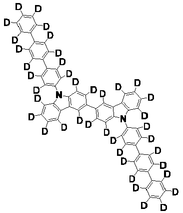 | 83% |
| 1-68 | 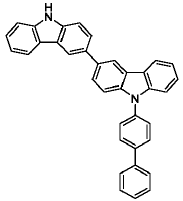 | 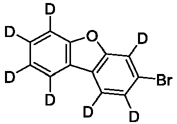 | 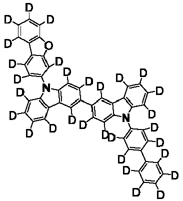 | 76% |

For reference, in a reaction of substituting hydrogen of an organic compound with deuterium, a substitution ratio of substituting from hydrogen to deuterium tends to increase and the yield tends to decrease as the reaction temperature is higher.

Considering the tendency, a reaction condition test experiment was conducted while progressing a D-substituted material synthesis method under conditions of the following Tables 4 and 5 in the preparation of Compound 1-26-1.

As a result, it was identified that Compound 1-26-1 was not synthesized under Conditions 1 to 4, and it was identified that the substitution ratio was the highest under Condition 7.

Accordingly, Compound 1-26-1 was synthesized under the reaction condition of Condition 7 having the highest substitution ratio.

**[Table 4]**

| Cond itio n | Compound (g, Equivalent) | Solvent (g) | Catalyst (mol%) | Temperat ure/Time (°C, d) | Reactor | Obtained Amount, Yield |
|---|---|---|---|---|---|---|
| 1 | 9H,9'H-3,3'-bicarbazole (1 g, 1 eq.) | D₂O (100 g) | Pt/C (10 mol%) | 150°C, 4d | Round Flask Under Ar Bag | NO Reaction |
| 2 | 9H,9'H-3,3'-bicarbazole (1 g, 1 eq.) | D₂O, i-PrOH, Cyclohexane (100 g, 50 g, 50 g) | Pt/C, Pd/C (10 mol%) | 150°C, 4d | Round Flask Under Ar Bag | NO Reaction |
| 3 | 9H,9'H-3,3'-bicarbazole (1 g, 1 eq.) | D₂O, i-PrOH, Cyclohexane (100 g, 50 g, 50 g) | Pt/C, Pd/C (10 mol%) | 200°C, 2d | Sealed Tube | NO Reaction |
| 4 | 9H,9'H-3,3'-bicarbazole (1 g, 1 eq.) | D₂O, i-PrOH, Cyclohexane | Pt/C, Pd/C (10 mol%) | 150°C, 4d | Sealed Tube | NO Reaction |
| | | (100 g, 50 g, 50 g) | | | | |

**[Table 5]**

| Conditi on | Compound (g, Equivalent) | Solvent (g, Equivalent) | Acid (g, Equivalent) | Tempera ture | Yield | Substitu tion ratio |
|---|---|---|---|---|---|---|
| 5 | 9H,9'H-3,3'-bicarbazole (1 g, 1 eq.) | Benzene-D6 (50 g, 139.9 eq.) | CF3SO3H (17 g, 25 eq.) | RT | 95% | 46% |
| 6 | 9H,9'H-3,3'-bicarbazole (1 g, 1 eq.) | Benzene-D6 (50 g, 139.9 eq.) | CF3SO3H (17 g, 25 eq.) | 30°C | 86% | 72% |
| 7 | 9H,9'H-3,3'-bicarbazole (1 g, 1 eq.) | Benzene-D6 (50 g, 139.9 eq.) | CF3SO3H (17 g, 25 eq.) | 40°C | 68% | 95% |
| 8 | 9H,9'H-3,3'-bicarbazole (1 g, 1 eq.) | DMSO-D6 (50 g, 139.9 eq.) | CF3SO3H (17 g, 25 eq.) | 40°C | 77% | 53% |
| 9 | 9H,9'H-3,3'-bicarbazole (1 g, 1 eq.) | DMF-D6 (50 g, 139.9 eq.) | CF3SO3H (17 g, 25 eq.) | 40°C | 79% | 62% |

In addition, considering the tendency that, in a reaction of substituting hydrogen of an organic compound with deuterium, a substitution ratio of substituting from hydrogen to deuterium tends to increase and the yield tends to decrease as the reaction temperature is higher in the case of Compound 1-50 as well, a reaction condition test experiment was conducted while progressing a D-substituted material synthesis method under conditions of the following Tables 6 and 7 in the preparation of Compound 1-50.

As a result, it was identified that Compound 1-50 was not synthesized under Conditions 1 to 4, and it was identified that the substitution ratio was the highest under Condition 7.

Accordingly, Compound 1-50 was synthesized under the reaction condition of Condition 7 having the highest substitution ratio.

**[Table 6]**

| Condi tion | Compound (g, Equivalent) | Solvent (g) | Catalyst (mol%) | Temperat ure/Time (°C, d) | Reactor | Obtained Amount, Yield |
|---|---|---|---|---|---|---|
| 1 | 1-50-1 (1 g, 1 eq.) | D₂O (100 g) | Pt/C (10 mol%) | 150°C, 4d | Round Flask Under Ar Bag | NO Reaction |
| 2 | 1-50-1 (1 g, 1 eq.) | D₂O, i-PrOH, Cyclohexane (100 g, 50 g, 50 g) | Pt/C, Pd/C (10 mol%) | 150°C, 4d | Round Flask Under Ar Bag | NO Reaction |
| 3 | 1-50-1 (1 g, 1 eq.) | D₂O, i-PrOH, Cyclohexane (100 g, 50 g, 50 g) | Pt/C, Pd/C (10 mol%) | 200°C, 2d | Sealed Tube | NO Reaction |
| 4 | 1-50-1 (1 g, 1 eq.) | D₂O, i-PrOH, Cyclohexane (100 g, 50 g, 50 g) | Pt/C, Pd/C (10 mol%) | 150°C, 4d | Sealed Tube | NO Reaction |

**[Table 7]**

| Condi tion | Compound (g, Equivalent) | Solvent (g, Equivalent) | Acid (g, Equivalent) | Tempe ratur e | Yiel d | Substit ution ratio |
|---|---|---|---|---|---|---|
| 5 | 1-50-1 (1 g, 1 eq.) | Benzene-D6 (50 g, 139.9 eq.) | CF3SO3H (17 g, 25 eq.) | RT | 93% | 42% |
| 6 | 1-50-1 (1 g, 1 eq.) | Benzene-D6 (50 g, 139.9 eq.) | CF3SO3H (17 g, 25 eq.) | 30°C | 83% | 82% |
| 7 | 1-50-1 (1 g, 1 eq.) | Benzene-D6 (50 g, 139.9 eq.) | CF3SO3H (17 g, 25 eq.) | 50°C | 78% | 98% |
| 8 | 1-50-1 (1 g, 1 eq.) | DMSO-D6 (50 g, 139.9 eq.) | CF3SO3H (17 g, 25 eq.) | 50°C | 88% | 59% |
| 9 | 1-50-1 (1 g, 1 eq.) | DMF-D6 (50 g, 139.9 eq.) | CF3SO3H (17 g, 25 eq.) | 50°C | 81% | 60% |

Herein, the substitution ratio is calculated as [(number of deuterium substituted after chemical reaction)/(number of hydrogen of compound before chemical reaction)]*100.

Compounds other than the compounds described in Preparation Example 1 to Preparation Example 3 and Table 1 to Table 3 were also prepared in the same manner as in the methods described in the preparation examples described above.

Synthesis identification results for the prepared compounds are shown in the following Table 8 and Table 9. The following Table 8 shows measurement values of ¹H NMR (CDCl₃, 300 Mz), and the following Table 9 shows measurement values of FD-mass spectrometry (FD-MS: field desorption mass spectrometry).

**[Table 8]**

| Compou nd | ¹H NMR (DMSO, 300 Mz) |
|---|---|
| 1-2 | δ=8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, m) |
| 1-3 | δ=8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, m) |
| 1-5 | δ=8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, m) |
| 1-6 | δ=8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, m) |
| 1-8 | δ=8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, m) |
| 1-19 | δ=8.55 (1H, d), 8.30 (1H, d), 8.13-8.19 (2H, m), 7.89-7.99 (4H, m), 7.77 (1H, d), 7.50-7.58 (2H, m), 7.35 (1H, t), 7.16-7.20 (2H, m) |
| 1-22 | δ=8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, m) |
| 1-26 | δ=7.91∼7.92 (8H, m), 7.75 (4H, d), 7.41∼7.49 (6H, m) |
| 1-27 | δ=8.21 (1H, s), 7.41-7.75 (13H, m) |
| 1-29 | δ=7.91∼7.94 (5H, m), 7.73∼7.75 (3H, m), 7.41∼7.62 (10H, m) |
| 1-32 | δ=8.21 (1H, s), 7.60∼7.75 (8H, m), 7.41∼7.49 (8H, m) |
| 1-33 | δ=8.21 (1H, s), 7.91∼7.92 (4H, m), 7.60∼7.75 (6H, m), 7.41∼7.49 (7H, m) |
| 1-36 | δ=8.21 (1H, s), 7.91∼7.92 (4H, m), 7.60∼7.75 (6H, m), 7.41∼7.49 (7H, m), 7.25 (4H, s) |
| 1-39 | δ=7.91∼7.92 (8H, m), 7.75 (4H, d), 7.41∼7.49 (6H, m), 7.25 (4H, s) |
| 1-44 | δ=7.91∼7.98 (6H, m), 7.75 (2H, d), 7.25∼7.54 (8H, m) |
| 1-50 | 5=no ¹H NMR peak due to deuterium content of 100% |
| 1-51 | 5=no ¹H NMR peak due to deuterium content of 100% |
| 1-53 | 5=no ¹H NMR peak due to deuterium content of 100% |
| 1-54 | 5=no ¹H NMR peak due to deuterium content of 100% |
| 1-56 | 5=no ¹H NMR peak due to deuterium content of 100% |
| 1-57 | 5=no ¹H NMR peak due to deuterium content of 100% |
| 1-58 | 5=no ¹H NMR peak due to deuterium content of 100% |
| 1-60 | 5=no ¹H NMR peak due to deuterium content of 100% |
| 1-62 | 5=no ¹H NMR peak due to deuterium content of 100% |
| 1-63 | 5=no ¹H NMR peak due to deuterium content of 100% |
| 1-67 | 5=no ¹H NMR peak due to deuterium content of 100% |
| 1-68 | 5=no ¹H NMR peak due to deuterium content of 100% |

**[Table 9]**

| Comp ound | FD-Mass | Comp ound | FD-Mass |
|---|---|---|---|
| 1-2 | m/z=654.91 (C48H14D18N2=654.37) | 1-3 | m/z=574.79 (C42H14D14N2=574.31) |
| 1-5 | m/z=654.91 (C48H14D18N2=654.37) | 1-6 | m/z=654.91 (C48H14D18N2=654.37) |
| 1-8 | m/z=654.91 (C48H14D18N2=654.37) | 1-19 | m/z=815.15 (C60H14D26N2=814.48) |
| 1-22 | m/z=815.15 (C606H14D26N2=814.48) | 1-26 | m/z=650.88 (C48H18D14N2=650.34) |
| 1-27 | m/z=574.79 (C42H14D14N2=574.31) | 1-29 | m/z=650.88 (C48H18D14N2=650.34) |
| 1-32 | m/z=650.88 (C48H18D14N2=650.34) | 1-33 | m/z=650.88 (C48H18D14N2=650.34) |
| 1-36 | m/z=726.98 (C54H22D14N2=726.38) | 1-39 | m/z=726.98 (C54H22D14N2=726.38) |
| 1-44 | m/z=664.87 (C48H16D14N2O=664.32) | 1-50 | m/z=668.99 (C48D32N2=668.46) |
| 1-51 | m/z=588.87 (C42D28N2=588.40) | 1-53 | m/z=668.99 (C48D32N2=668.46) |
| 1-54 | m/z=668.99 (C48D32N2=668.46) | 1-56 | m/z=668.99 (C48D32N2=668.46) |
| 1-57 | m/z=668.99 (C48D32N2=668.46) | 1-58 | m/z=749.12 (C54D36N2=748.51) |
| 1-60 | m/z=749.12 (C54D36N2=748.51) | 1-62 | m/z=749.12 (C54D36N2=748.51) |
| 1-63 | m/z=749.12 (C54D36N2=748.51) | 1-67 | m/z=829.24 (C60D40N2=828.57) |
| 1-68 | m/z=680.96 (C48D30N2O=680.42) | | |

### <Experimental Example>

### (1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, the compound represented by Chemical Formula 1 of the present disclosure described in the following Table 10 was deposited to 400 Å as a host, and a green phosphorescent dopant [Ir(ppy)₃] was doped by 7% of the deposited thickness of the light emitting layer and deposited. After that, BCP (bathocuproine) was deposited to a thickness of 60 Å as a hole blocking layer, and Alq₃ was deposited to a thickness of 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured (Examples 1 to 22 and Comparative Examples 1 to 5) .

Meanwhile, all the organic compounds required to manufacture the organic light emitting device were vacuum sublimation purified under 10⁻⁸ torr to 10⁻⁶ torr for each material to be used in the organic light emitting device manufacture.

### (2) Driving Voltage and Light Emission Efficiency of Organic Light Emitting Device

For each of the organic light emitting devices of Examples 1 to 22 and Comparative Examples 1 to 5 manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc.. Herein, T₉₀ means a lifetime (unit: h, time) taken to become 90% with respect to initial luminance.

Results of measuring driving voltage, light emission efficiency, color (EL color) and lifetime of the organic light emitting devices manufactured according to the present disclosure are as shown in the following Table 10.

**[Table 10]**

| | Compound | Driving Voltage (V) | Efficiency (cd/A) | Color Coordinate (x, y) | Lifetime (T₉₀) |
|---|---|---|---|---|---|
| Comparative Example 1 | A | 6.11 | 44.1 | Green | 59 |
| Comparative Example 2 | B | 6.15 | 45.5 | | 62 |
| Comparative Example 3 | C | 6.23 | 43.6 | | 68 |
| Comparative Example 4 | D | 6.48 | 42.9 | | 60 |
| Comparative Example 5 | E | 6.90 | 40.7 | | 50 |
| Example 1 | 1-2 | 5.68 | 55.4 | | 90 |
| Example 2 | 1-4 | 5.34 | 54.8 | | 84 |
| Example 3 | 1-5 | 5.48 | 53.9 | | 88 |
| Example 4 | 1-7 | 5.59 | 56.0 | | 80 |
| Example 5 | 1-8 | 5.61 | 55.8 | | 86 |
| Example 6 | 1-9 | 5.79 | 53.7 | | 88 |
| Example 7 | 1-26 | 3.71 | 74.6 | | 130 |
| Example 8 | 1-28 | 3.60 | 75.0 | | 124 |
| Example 9 | 1-29 | 3.88 | 68.7 | | 128 |
| Example 10 | 1-31 | 3.92 | 69.1 | | 120 |
| Example 11 | 1-32 | 3.67 | 70.5 | | 126 |
| Example 12 | 1-33 | 3.83 | 71.3 | | 128 |
| Example 13 | 1-50 | 4.03 | 70.3 | | 150 |
| Example 14 | 1-51 | 4.28 | 74.7 | | 146 |
| Example 15 | 1-52 | 4.16 | 75.1 | | 142 |
| Example 16 | 1-53 | 4.26 | 79.6 | | 148 |
| Example 17 | 1-55 | 4.33 | 77.5 | | 138 |
| Example 18 | 1-56 | 4.22 | 78.6 | | 144 |
| Example 19 | 1-57 | 4.50 | 73.2 | | 148 |
| Example 20 | 1-73 | 4.76 | 74.6 | | 82 |
| Example 21 | 1-74 | 4.71 | 75.0 | | 122 |
| Example 22 | 1-75 | 4.82 | 73.7 | | 140 |

Compounds used in Comparative Examples 1 to 5 of Table 10 are as follows.

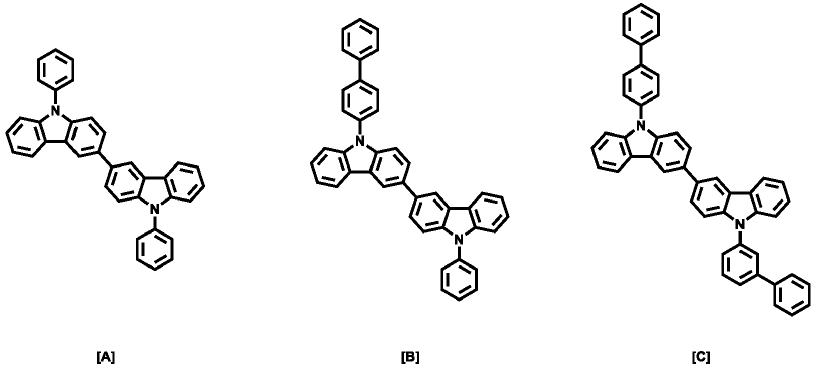

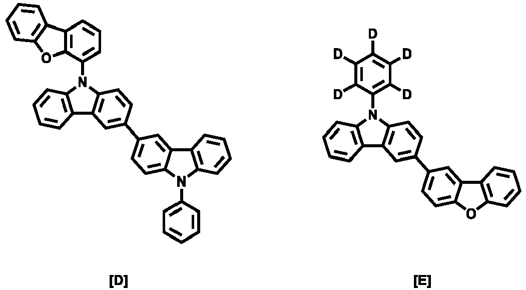

As seen from the results of Table 10, the organic light emitting device using the organic light emitting device light emitting layer material of the present application had lower driving voltage, enhanced light emission efficiency and significantly improved lifetime compared to Comparative Examples 1 to 5.

The compound according to the present application is a compound substituted with deuterium, and the compound of the comparative example is a compound either substituted with hydrogen or that is not biscarbazole. The compound substituted with deuterium having twice the atomic mass compared to hydrogen has decreased energy in a ground state and weakened intermolecular interactions due to lower zero-point energy and vibrational energy compared to the compound bonding to hydrogen, and as a result, a thin film may be made in a non-crystalline state and a device lifetime may be enhanced.

The deuterium-substituted compound has enhanced stability by having low energy in a ground state, has enhanced molecular stability since dissociation energy of C-D is high, and the lifetime may be enhanced.

Specifically, it was identified that deuterium-substituted Compound 1-51 corresponding to the compound according to the present application had enhanced driving and lifetime due to molecular stability and non-crystallinity compared to hydrogen-substituted Compounds A, B, C and D of the comparative examples, and was able to facilitate energy transfer to the dopant by minimizing energy loss due to low vibrational energy, and enhance efficiency.

It was identified that Compound E of the comparative example had dibenzofuran substituting the No. 3 position of carbazole unlike biscarbazole, and had low efficiency, driving and lifetime compared to the compound corresponding to the compound according to the present application due to low HOMO and low hole stability.

Compounds 1-2, 1-6 and 1-50 corresponding to the compound according to the present application was able to enhance efficiency by reducing energy loss due to low vibrational energy, and was able to enhance the lifetime by lowering energy in a ground state.

Particularly, it was identified that Compound 1-50 having the whole compound substituted with deuterium was able to enhance device efficiency and lifetime compared to Compound 1-26 having only biscarbazole substituted with deuterium and Compound 1-2 having only the aryl group (corresponding to R32 and R33 of Chemical Formula 1) substituted with deuterium.

This may be considered to be due to the fact that intermolecular interactions increase as the deuterium content increases, which results in shorter intermolecular distances and improved mobility.

In addition, when comparing Compounds 1-5 and 1-29 corresponding to the compounding according to the present application, it was identified that Compound 1-29 resulted in enhanced device efficiency and lifetime compared to Compound 1-5.

Specifically, Compounds 1-5 and 1-29 corresponding to the compound according to the present application are different in that they are, as described above, a compound having only biscarbazole substituted with deuterium (Compound 1-29) and a compound having only the aryl group substituted with deuterium (Compound 1-5).

Generally, when a radical cation is produced in carbazole, the No. 3 position of the carbazole is activated to form a dimer inhibiting efficiency and lifetime of a device.

However, Compound 1-29 corresponding to the compound according to the present application having biscarbazole substituted with deuterium may effectively stabilize the radical cation and effectively enhance the lifetime as a result, and Compound 1-5 having the aryl group substituted with deuterium is not able to contribute to stabilization of the radical cation of biscarbazole.

Accordingly, although the device using deuterium-substituted Compound 1-5 had an enhanced lifetime, it was identified that the device using Compound 1-29 having only biscarbazole substituted with deuterium had a more enhanced lifetime.

In other words, when the compound represented by Chemical Formula 1 is partially substituted with deuterium, performance of the device is more superior when hydrogen of the biscarbazole of Chemical Formula 1 is substituted with deuterium compared to when hydrogen of the aryl group of Chemical Formula 1 is substituted with deuterium (comparison between Compound 1-5 and Compound 1-29), and performance of the device is more superior as the deuterium substitution ratio increases (comparison between Compounds 1-2, 1-26 and 1-50) .

For reference, the No. 3 position of carbazole herein means the No. 3 position of the following Chemical Formula A.

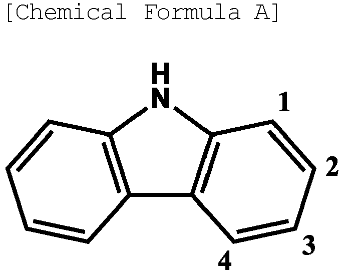

In other words, due to its structural properties, the compound according to the present application is effective in lowering a driving voltage, enhancing light emission efficiency and significantly improving a lifetime of an organic light emitting device using the compound as a material of an organic material layer. Particularly, the effect is more superior when using the compound according to the present application as a material of a light emitting layer.

### <Reference Numeral>

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

## Claims

1. A heterocyclic compound represented by the following Chemical Formula 1: 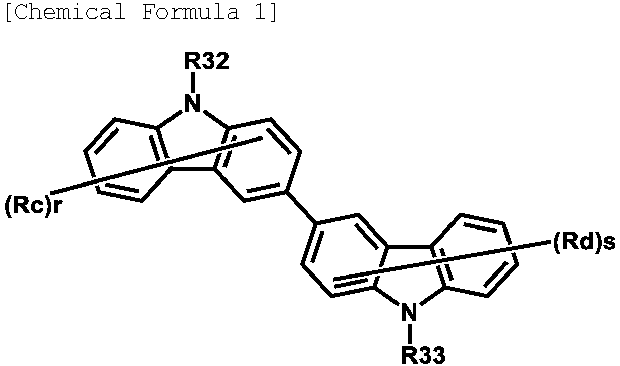 wherein, in Chemical Formula 1,
Rc and Rd are the same as or different from each other, and each independently selected from the group consisting of hydrogen; deuterium; halogen; a cyano group; a substituted or unsubstituted alkyl group having 1 to 60 carbon atoms; a substituted or unsubstituted alkenyl group having 2 to 60 carbon atoms; a substituted or unsubstituted alkynyl group having 2 to 60 carbon atoms; a substituted or unsubstituted alkoxy group having 2 to 60 carbon atoms; a substituted or unsubstituted cycloalkyl group having 3 to 60 carbon atoms; a substituted or unsubstituted heterocycloalkyl group having 2 to 60 carbon atoms; a substituted or unsubstituted aryl group having 6 to 60 carbon atoms; a substituted or unsubstituted heteroaryl group having 2 to 60 carbon atoms; a substituted or unsubstituted phosphine oxide group; and a substituted or unsubstituted amine group, r and s are an integer of 0 to 7, and when r is 2 or greater, Res are the same as or different from each other, and when s is 2 or greater, Rds are the same as or different from each other;
R32 and R33 are the same as or different from each other, and each independently a substituted or unsubstituted silyl group; a substituted or unsubstituted aryl group having 6 to 60 carbon atoms; or a substituted or unsubstituted heteroaryl group having 2 to 60 carbon atoms; and
the heterocyclic compound represented by Chemical Formula 1 has a deuterium content of greater than 0% and less than or equal to 100%.

2. The heterocyclic compound of Claim 1, wherein the heterocyclic compound represented by Chemical Formula 1 has a deuterium content of greater than or equal to 40% and less than or equal to 100%.

3. The heterocyclic compound of Claim 1, wherein the deuterium content is greater than or equal to 0% and less than or equal to 100% based on the positions where Rc and Rd are substituted, and when the deuterium content is 0% based on the positions where Rc and Rd are substituted, at least one of R32 and R33 has a deuterium content of greater than 0% and less than or equal to 100%.

4. The heterocyclic compound of Claim 1, wherein the deuterium content is greater than or equal to 0% and less than or equal to 100% based on the position where Rc is substituted, the deuterium content is greater than or equal to 0% and less than or equal to 100% based on the position where Rd is substituted, and when each deuterium content is 0% based on the positions where Rc and Rd are substituted, at least one of R32 and R33 has a deuterium content of greater than 0% and less than or equal to 100%.

5. The heterocyclic compound of Claim 1, wherein r and s are an integer of 2 to 7, and when r is 2 or greater, Res are the same as or different from each other, and when s is 2 or greater, Rds are the same as or different from each other, and two or more of Res are deuterium and two or more of Rds are deuterium.

6. The heterocyclic compound of Claim 5, wherein R32 and R33 have a deuterium content of 0%.

7. The heterocyclic compound of Claim 5, wherein R32 and R33 are the same as or different from each other and each independently a substituted or unsubstituted silyl group; a substituted or unsubstituted aryl group having 6 to 40 carbon atoms; or a substituted or unsubstituted heteroaryl group having 2 to 40 carbon atoms, and R32 and R33 have a deuterium content of 0%.

8. The heterocyclic compound of Claim 1, wherein Chemical Formula 1 is represented by any one of the following compounds:
| | | | |
|---|---|---|---|
| 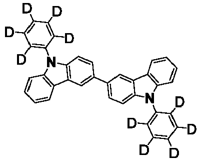 | 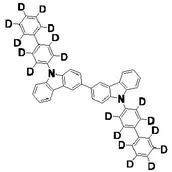 | 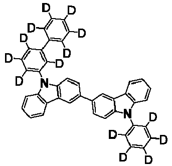 | 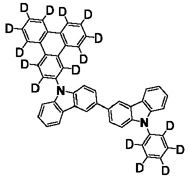 |
| 1-1 | 1-2 | 1-3 | 1-4 |
| 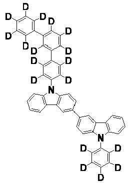 | 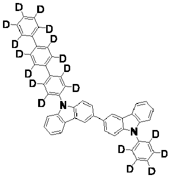 | 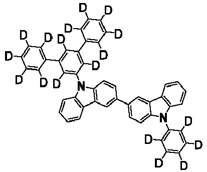 | 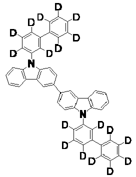 |
| 1-5 | 1-6 | 1-7 | 1-8 |
| 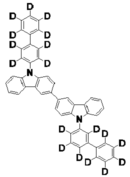 | 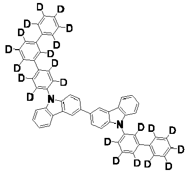 | 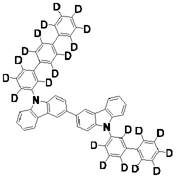 | 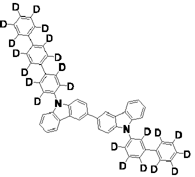 |
| 1-9 | 1-10 | 1-11 | 1-12 |
| 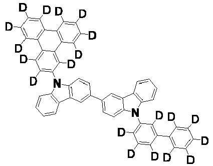 | 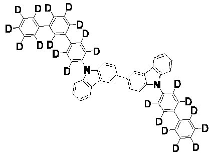 | 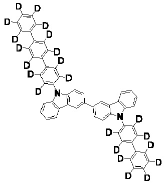 | 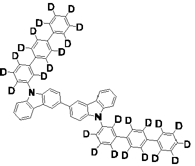 |
| 1-13 | 1-14 | 1-15 | 1-16 |
| 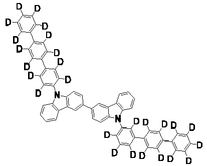 | 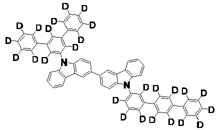 | 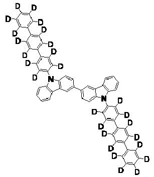 | 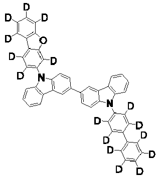 |
| 1-17 | 1-18 | 1-19 | 1-20 |
| 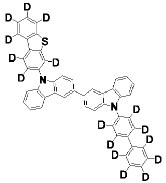 | 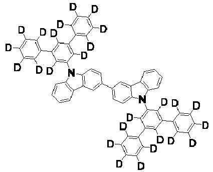 | 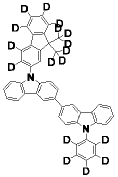 | 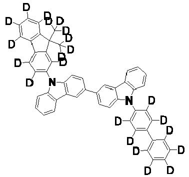 |
| 1-21 | 1-22 | 1-23 | 1-24 |
| 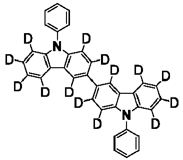 | 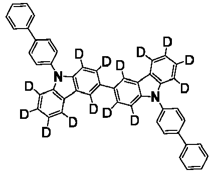 | 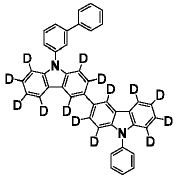 | 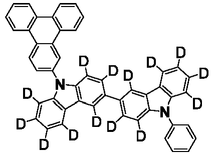 |
| 1-25 | 1-26 | 1-27 | 1-28 |
| 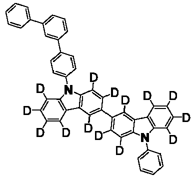 | 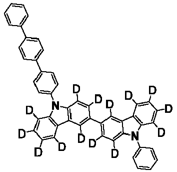 | 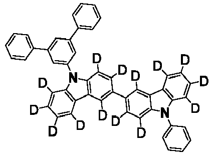 | 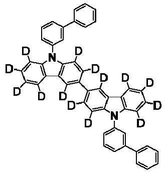 |
| 1-29 | 1-30 | 1-31 | 1-32 |
| 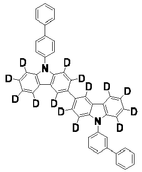 | 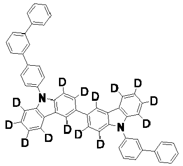 | 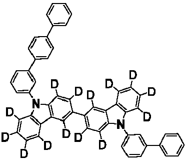 | 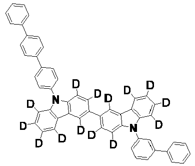 |
| 1-33 | 1-34 | 1-35 | 1-36 |
| 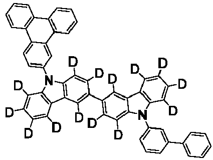 | 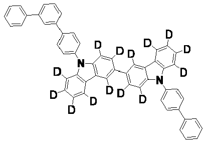 | 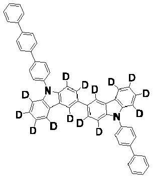 | 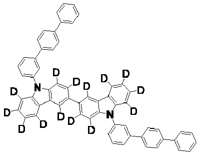 |
| 1-37 | 1-38 | 1-39 | 1-40 |
| 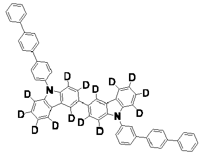 | 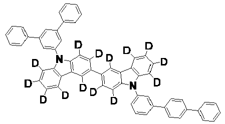 | 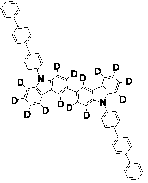 | 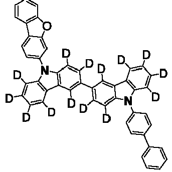 |
| 1-41 | 1-42 | 1-43 | 1-44 |
| 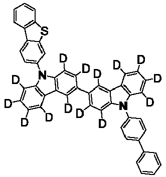 | 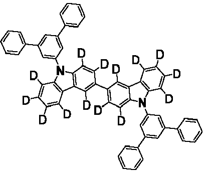 | 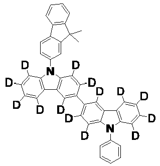 | 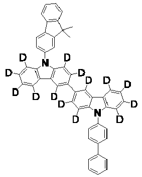 |
| 1-45 | 1-46 | 1-47 | 1-48 |
| 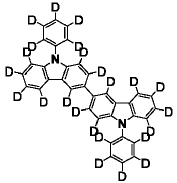 | 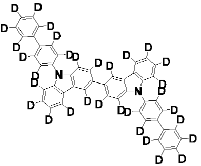 | 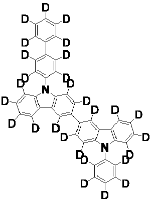 | 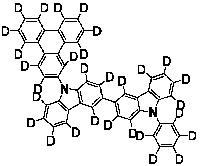 |
| 1-49 | 1-50 | 1-51 | 1-52 |
| 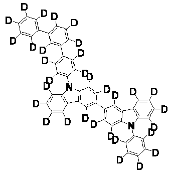 | 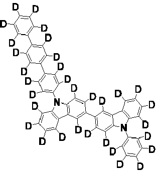 | 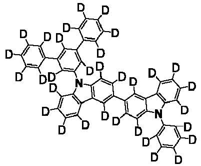 | 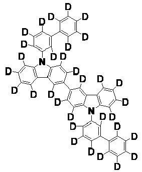 |
| 1-53 | 1-54 | 1-55 | 1-56 |
| 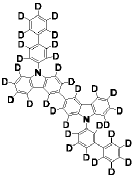 | 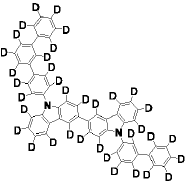 | 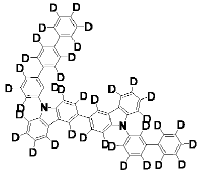 | 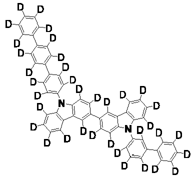 |
| 1-57 | 1-58 | 1-59 | 1-60 |
| 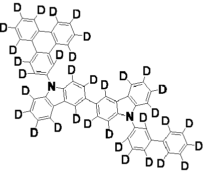 | 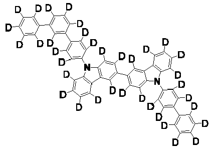 | 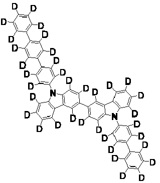 | 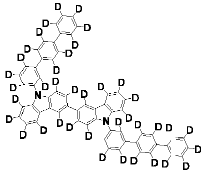 |
| 1-61 | 1-62 | 1-63 | 1-64 |
| 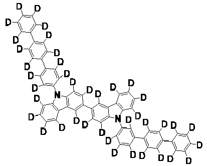 | 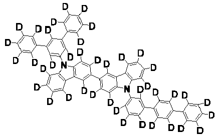 | 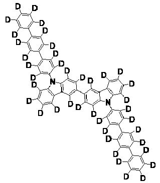 | 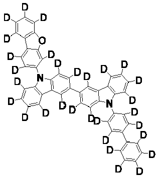 |
| 1-65 | 1-66 | 1-67 | 1-68 |
| 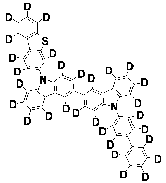 | 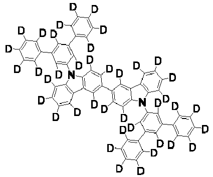 | 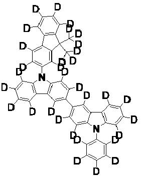 | 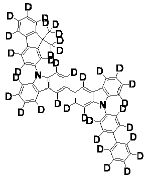 |
| 1-69 | 1-70 | 1-71 | 1-72 |
| 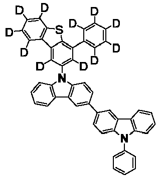 | 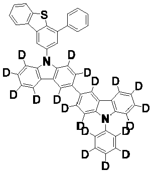 | 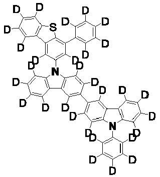 | 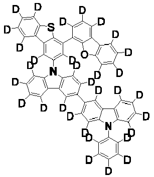 |
| 1-73 | 1-74 | 1-75 | 1-76 |

9. An organic light emitting device comprising:
a first electrode;
a second electrode; and
one or more organic material layers provided between the first electrode and the second electrode,
wherein one or more layers of the organic material layers comprise the heterocyclic compound of any one of Claims 1 to 8.

10. The organic light emitting device of Claim 9, wherein the organic material layer comprises one or more light emitting layers, and the light emitting layer comprises the heterocyclic compound.

11. The organic light emitting device of Claim 10, wherein the light emitting layer comprises a host material, and the host material comprises the heterocyclic compound.

12. The organic light emitting device of Claim 9, further comprising one, two or more layers selected from the group consisting of a light emitting layer, a hole injection layer, a hole transfer layer, an electron injection layer, an electron transfer layer, a hole auxiliary layer and a hole blocking layer.
